# EUROPEAN PATENT APPLICATION

(11) **EP 1 914 227 A1**
(43) Date of publication of application: **23.04.2008**
(21) Application number: 06119895.8
(22) Date of filing: 31.08.2006
(51) Int. Cl.: C07D 215/26, A61K 31/4704, A61P 11/00

(54) **Polymorphic crystal form of a indan-2-ylamino-hydroxyethyl-quinolinone maleate derivative as beta-adrenoceptor agonist**

(71) Applicant: Novartis AG, 4056 Basel (CH)
(72) Inventor: The designation of the inventor has not yet been filed
(74) Representative: McLean, Craig Sutherland

(57) **Abstract**

New polymorphic crystal form of (R)-5-[2-(5,6-diethyl-indan-2-ylamino)-1-hydroxyethyl]-8-hydroxy-1 H-quinolin-2-one maleate designated crystal form Qalpha that is useful in the treatment of inflammatory or obstructive airways diseases. A method for preparing crystal form Qalpha is also described.

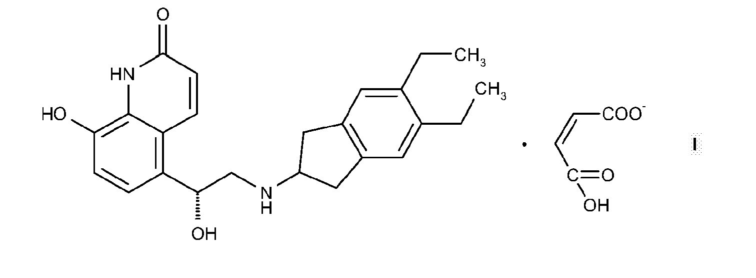

## Description

This invention relates to a new polymorphic crystal form of (R)-5-[2-(5,6-diethyl-indan-2-ylamino)-1-hydroxyethyl]-8-hydroxy-1H-quinolin-2-one maleate designated crystal form Qalpha and methods for preparing same.

The compound (R)-5-[2-(5,6-diethyl-indan-2-ylamino)-l-hydroxyethyl]-8-hydroxy-1H-quinolin-2-one maleate has the chemical structure of formula I 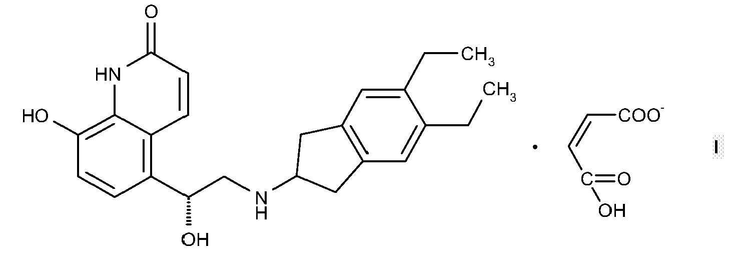 (R)-5 -[2-(5 ,6-diethyl-indan-2-ylamino)-1-hydroxyethyl]-8-hydroxy-1H-quinolin-2-one maleate, herein the "compound of formula I" is a potent beta-2 adrenoceptor agonist and effective bronchodilator. Its rapid onset of action and prolonged stimulating action on the β₂-adrenoceptor, e.g. for 24 hours or longer, means it is especially suitable for the treatment of respiratory diseases such as asthma and chronic obstructive pulmonary disease (COPD).

The compound of formula I is prepared by the processes described in international patent applications WO 2000/75114 and WO 2005/123684, the contents of which are incorporated herein by reference.

This compound has been investigated for use as a pharmaceutical. The existence of various crystallisation polymorphic forms of the compound has been explored in order to determine the most appropriate form of the compound for the proposed use.

A novel crystal form of the compound of formula I has now been isolated and designated crystal form Qalpha. This crystal form has very good stability, facilitating its use in the preparation of pharmaceutical dosage forms.

Accordingly, the present invention provides in one aspect crystalline (R)-5-[2-(5,6-diethyl-indan-2-ylamino)-1-hydroxyethyl]-8-hydroxy-1H-quinolin-2-one maleate designated crystal form Qalpha.

In a second aspect the invention provides crystalline (R)-5-[2-(5,6-diethyl-indan-2-ylamino)-1-hydroxyethyl]-8-hydroxy-1H-quinolin-2-one maleate designated crystal form Qalpha characterised by a melting point, by Differential Scanning Calorimetry, of about 192°C with simultaneous decomposition.

In a third aspect the invention provides crystalline (R)-5-[2-(5,6-diethyl-indan-2-ylamino)-1-hydroxyethyl]-8-hydroxy-1H-quinolin-2-one maleate in crystal form Qalpha having the following characteristic diffraction lines (20 in angular degrees ± 0.2°) in the X-ray diffraction pattern thereof: 5.3 °, 10.1 °, 12.7 °, 16.4 °, 20.0 ° and 25.8°.

In a fourth aspect the invention provides a pharmaceutical composition comprising, as active ingredient, an effective amount of crystalline (R)-5-[2-(5,6-diethyl-indan-2-ylamino)-1-hydroxyethyl]-8-hydroxy-1H-quinolin-2-one maleate, optionally together with a pharmaceutically acceptable carrier. Preferably the composition is in inhalable form.

In a fifth aspect the invention concerns the use of crystalline (R)-5-[2-(5,6-diethyl-indan-2-ylamino)-1-hydroxyethyl]-8-hydroxy-1H-quinolin-2-one maleate for the preparation of a medicament for the treatment of an inflammatory or obstructive airways disease.

In a sixth aspect the invention provides a method of preparing crystalline (R)-5-[2-(5,6-diethyl-indan-2-ylamino)-1-hydroxyethyl]-8-hydroxy-1H-quinolin-2-one maleate which comprises crystallising (R)-5-[2-(5,6-diethyl-indan-2-ylamino)-1-hydroxyethyl]-8-hydroxy-1H-quinolin-2-one maleate from a solution thereof in ethanol ALI (90% ethanol, 5% water, 5% isopropanol).

Terms used in the specification has the following meanings:
"Polymorphism" as used herein is the ability of a compound to crystallize into more than one distinct crystal species. Polymorphs (or crystalline modifications) have an identical chemical structure but often quite different physicochemical properties. Polymorphs include enantiotropic polymorphs and monotropic polymorphs.
"Amorphous" as used herein described a disordered solid state, which may appear during manufacture of the drug substance (crystallization step, drying, milling) or the drug product (granulation, compression). The X-ray powder diffraction pattern of an amorphous solid exhibits no sharp peaks.
"Pseudopolymorph" is a solvate or hydrate of a compound that, like polymorphs, have different physical properties, however, unlike polymorphs, have a different chemical composition. In other words it is a crystal form that incorporates either stoichiometric of non-stoichiometric amounts of a water (in the case of a hydrate) or another solvent (in the case of a solvate).
"Crystallographically pure" as used herein in relation to a crystal form means the crystal form contains at most about 1 % (w/w) of another form. Thus e.g. "crystallographically pure crystal form Qalpha" contains ≤ about 1 % (w/w) of another form.

Throughout this specification and in the claims that follow, unless the context requires otherwise, the word "comprise", or variations such as "comprises" or "comprising", will be understood to imply the inclusion of a stated integer or group of integers but not the exclusion of any other integer or group of integers.

The new polymorphic crystal form of the present invention shall now be described with reference to the accompanying drawings. In the drawings:
Figure 1 is an X-ray diffraction pattern for crystal form Qalpha.
Figure 2 is an X-ray diffraction pattern for amorphous (R)-5-[2-(5,6-diethyl-indan-2-ylamino)-1-hydroxyethyl]-8-hydroxy-1H-quinolin-2-one maleate.
Figure 3 is a Raman spectrum for crystal form Qalpha.
Figure 4 is a Raman spectrum for amorphous (R)-5-[2-(5,6-diethyl-indan-2-ylamino)-1-hydroxyethyl]-8-hydroxy-1H-quinolin-2-one maleate.
Figure 5 is an IR spectrum for crystal form Qalpha.
Figure 6 is an IR spectrum for amorphous (R)-5-[2-(5,6-diethyl-indan-2-ylamino)-1-hydroxyethyl]-8-hydroxy-1H-quinolin-2-one maleate.

The present invention provides crystalline (R)-5-[2-(5,6-diethyl-indan-2-ylamino)-1-hydroxyethyl]-8-hydroxy-1H-quinolin-2-one maleate designated crystal form Qalpha.

The compound of formula I in its partially amorphous form may be prepared in accordance with the method given in Example 21 of international patent application WO 2000/75114. This involves reacting (R)-8-benzyloxy-5-oxiranylcarbostyril with 5,6-diethyl-indan-2-ylamine to give 8-benzyloxy-5-[(R)-2-(5,6-diethyl-indan-2-ylamino)-1-hydroxy-ethyl]-1H-quinolin-2-one, subjecting the latter to a deprotecting reaction to replace the benzyl group by hydrogen, and recovering the resultant compound as a maleate salt. (R)-8-benzyloxy-5-oxiranyl-carbostyril may be prepared as described in W0 1995/25104. 5,6-Diethyl-indan-2-ylamine may be prepared as described in WO 2003/76387.

However the compound of formula I is preferably prepared in accordance with the method described in international patent application WO 2005/123684, wherein the aforementioned 8-benzyloxy-5-(R)-oxiranyl-(1H)-quinolin-2-one intermediate is prepared by an asymmetric hydrogenation process.

While the first recovery of the compound of formula I in crystalline form occurred several years after the first synthesis of the compound, initially obtained only in amorphous form, it has now been found since its first crystallization that the compound could be induced to crystallize from the amorphous form quite readily. The crystalline material has thus now become easily accessible, using a variety of experimental conditions extending beyond the initially used recrystallization conditions, which involved the addition of water to an ethanolic solution of the amorphous compound.

Crystal form Qalpha may be prepared by crystallising the compound of formula I from a solution thereof in ethanol ALI (90% ethanol, 5% water, 5% isopropanol), for example by equilibrating the compound in that solvent over 3 days at 25° C ± 01, or analogously such as hereinafter described in Example 1. The crystallisation may be induced by, for example, cooling a supersaturated solution of the compound of formula I in the solvent, or by adding to the solution of the compound of formula I a solvent in which the compound of formula I is less soluble. The starting solution of the compound of formula I may be at ambient or elevated (up to reflux) temperature.

It is preferred to add "seeds" of crystalline material to the solution in order to induce crystallization.

The compound of formula I in crystalline form can readily be isolated, it can e.g. be filtered off or centrifuged from the crystallization medium,

For the preparation of crystal form Qalpha working up may be carried out generally using known procedures for the separation of the crystallisate from the mother liquor, for example by filtration, with or without the assistance of pressure and/or vacuum, or by centrifugation, and subsequent drying of the crystallisate.

Amorphous parts can be converted into the crystalline form by suitable art-known methods.

Crystal form Qalpha can be characterised in a variety of ways.

Crystal form Qalpha has a melting point, by Differential Scanning Calorimetry, of about 192°C with simultaneous decomposition, for example at a heating rate of 10 K/min.

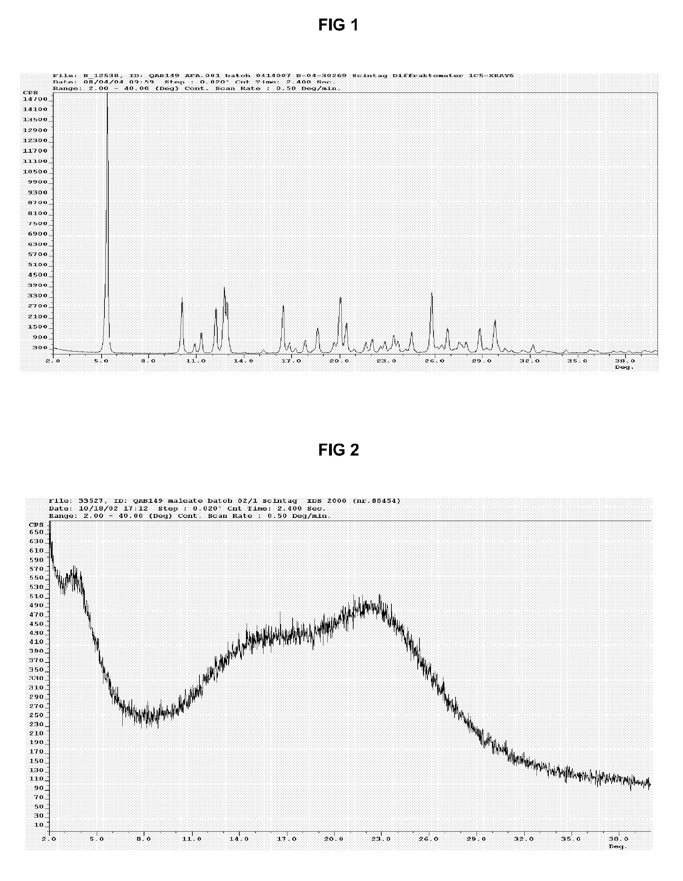

Crystal form Qalpha has the characteristic diffraction lines (20 in angular degrees ± 0.2°) in the X-ray diffraction pattern thereof shown in Figure 1. This is described in more detail in Example 2. The XRPD pattern shows characteristic diffraction lines (20 in angular degrees ± 0.2°) at 5.3 °, 10.1 °, 12.2 °, 12.7 °, 12.9 °, 16.4 °, 20.0 °, 23.4 °, 24.5 °, 25.8°, 26.8 °, 28.8° and 29.7°. The peaks at 5.3 °, 10.1 °, 12.7 °, 16.4 °, 20.0 ° and 25.8° are the predominant peaks. The strongest diffraction peak is at 5.3°. The X-ray diffraction pattern for amorphous (R)-5-[2-(5,6-diethyl-indan-2-ylamino)-1-hydroxyethyl]-8-hydroxy-1H-quinolin-2-one maleate is shown in Figure 2 and is clearly distinguishable from the XRPD pattern of crystal form Qalpha.

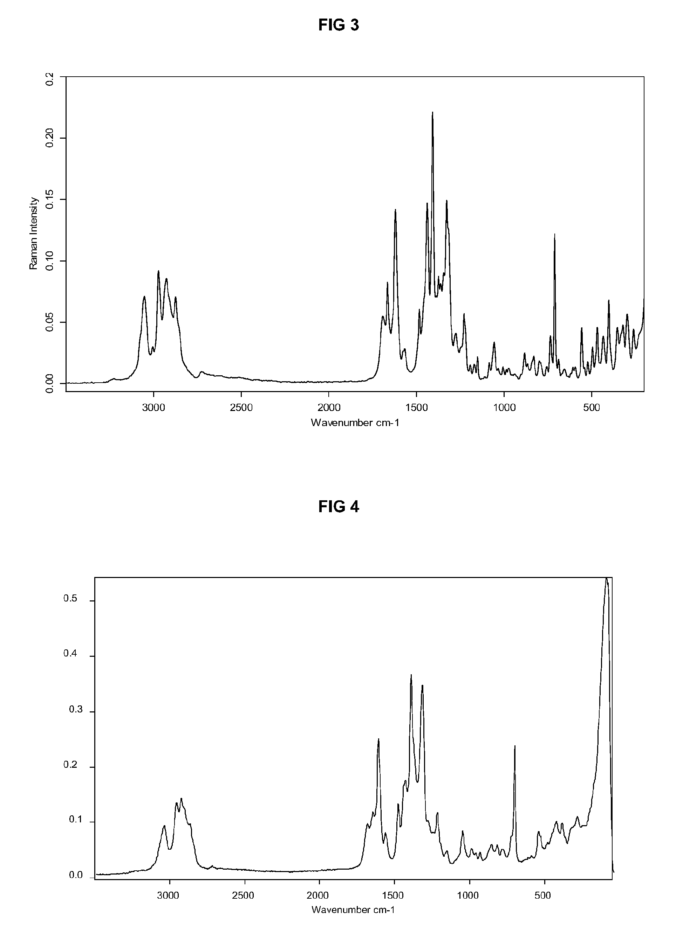

Crystal form Qalpha can be characterised by its FT-Raman spectrum. The FT-Raman spectrum of crystal form Qalpha is shown in Figure 3. This is described in more detail in Example 3. The FT-Raman spectrum for amorphous (R)-5-[2-(5,6-diethyl-indan-2-ylamino)-1-hydroxy-ethyl]-8-hydroxy-1H-quinolin-2-one maleate is shown in Figure 4 and is clearly distinguishable from the FT-Raman spectrum of crystal form Qalpha.

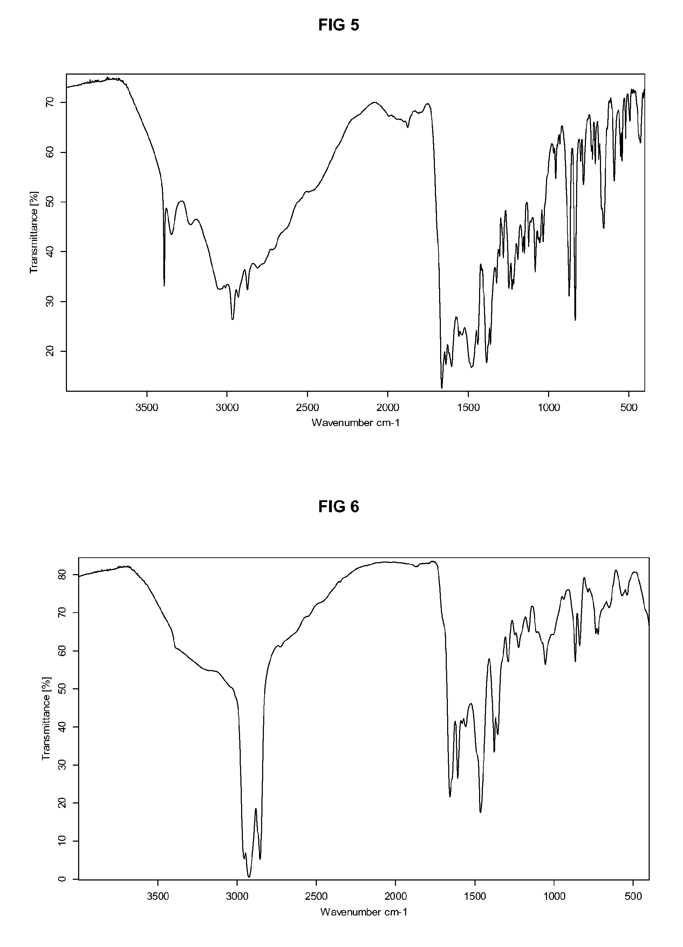

Crystal form Qalpha can be characterised by its FT-IR spectrum. The FT-IR spectrum of crystal form Qalpha is shown in Figure 5. This is described in more detail in Example 4. The FT-IR spectrum for amorphous (R)-5-[2-(5,6-diethyl-indan-2-ylamino)-1-hydroxyethyl]-8-hydroxy-1H-quinolin-2-one maleate is shown in Figure 6 and is clearly distinguishable from the FT-IR spectrum of crystal form Qalpha.

Crystal form Qalpha can be characterised by its crystallographic data as described in Example 5 and by its crystal packing as described in Example 6.

Samples of crystal form Qalpha observed by scanning electron microscopy (SEM) reveal a population of large needle-shaped crystals.

A second crystal form of the compound of formula I, namely qbeta, has been obtained by crystallising the compound from a solution thereof in tetrahydrofuran (THF) in water (1/1 V/V) at 80° C ± 01, but this is not pure. It contains crystal form Qalpha. Crystal form qbeta was also obtained on one occasion by crystallising the compound from the same solution at 25° C ± 01, as indicated by the presence of a small peak at 3.8° in the XRPD pattern. However it was not possible to reproduce this. A strong similitude of X-ray diffraction pattern was observed with an anhydrous racemic compound, which suggests that crystal form qbeta is a hydrate form of the racemic compound i.e. a pseudopolymorph.

Given its anti-inflammatory activity, the compound of formula I in crystal form Qalpha is useful in the treatment of inflammatory conditions, particularly inflammatory or obstructive airways diseases. Treatment in accordance with the invention may be symptomatic or prophylactic.

Inflammatory or obstructive airways diseases to which the present invention is applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant from any previously administered symptomatic asthma therapy.

Other inflammatory or obstructive airways diseases and conditions to which the present invention is applicable include acute lung injury (ALI), adult respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular other inhaled drug therapy. The invention is also applicable to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Having regard to its anti-inflammatory activity, in particular in relation to inhibition of eosinophil activation, the compound of formula I in crystal form Qalpha is also useful in the treatment of eosinophil related disorders, e.g. eosinophilia, in particular eosinophil related disorders of the airways (e.g. involving morbid eosinophilic infiltration of pulmonary tissues) including hypereosinophilia as it effects the airways and/or lungs as well as, for example, eosinophil-related disorders of the airways consequential or concomitant to Löffler's syndrome, eosinophilic pneumonia, parasitic (in particular metazoan) infestation (including tropical eosinophilia), bronchopulmonary aspergillosis, polyarteritis nodosa (including Churg-Strauss syndrome), eosinophilic granuloma and eosinophil-related disorders affecting the airways occasioned by drug-reaction.

The compound of formula I in crystal form Qalpha is also useful in the treatment of inflammatory conditions of the skin, for example psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, pemphisus, epidermolysis bullosa acquisita, and other inflammatory conditions of the skin.

The compound of formula I in crystal form Qalpha may also be used for the treatment of other diseases or conditions, in particular diseases or conditions having an inflammatory component, for example, treatment of diseases and conditions of the eye such as conjunctivitis, kerato-conjunctivitis sicca, and vernal conjunctivitis, diseases affecting the nose including allergic rhinitis, diseases of the joints such as rheumatoid arthritis and inflammatory bowel disease such as ulcerative colitis and Crohn's disease.

Further, the compound of formula I in crystal form Qalpha may also be used for the treatment of cystic fibrosis, pulmonary hypertension and pulmonary fibrosis.

The compound of formula I in crystal form Qalpha is also useful as a co-therapeutic agent for use in conjunction with other drug substances for treatment of airways diseases, particularly anti-inflammatory, bronchodilatory, antihistaminic/anti-allergic or anti-tussive drug substances, particularly in the treatment of obstructive or inflammatory airways diseases such as those mentioned hereinbefore, for example as potentiators of therapeutic activity of such drugs or as a means of reducing required dosaging or potential side effects of such drugs. The compound of formula I in crystal form Qalpha may be mixed with the other drug in a fixed pharmaceutical composition or it may be administered separately, before, simultaneously with or after the other drug.

Such anti-inflammatory drugs include steroids, in particular glucocorticosteroids such as budesonide, beclamethasone dipropionate, fluticasone propionate, ciclesonide, dexamethasone, flunisolide, mometasone furoate and triamcinolone but also compounds described in WO 02/00679, WO 02/88167, WO 02/12266 and WO 02/100879 (including salts or derivatives thereof such as sodium salts, sulphobenzoates, phosphates, isonicotinates, acetates, propionates, dihydrogen phosphates, palmitates, pivalates or furoates, and, where possible, hydrates); dopamine agonists such as bromocriptine, cabergolin, alpha- dihydroergocryptine, lisuride, pergolide, pramipexol, roxindol, ropinirol, talipexol, tergurid and viozan (including pharmaceutically acceptable salts thereof such as salts of hydrochloric acid, hydrobromic acid, sulphuric acid, phosphoric acid, methanesulphonic acid, acetic acid, fumaric acid, succinic acid, lactic acid, citric acid, tartaric acid and maleic acid); LTB4 antagonists such as those described in US 5451700; LTB4 antagonists such as those described in US 5451700; LTD4 antagonists such as montelukast and zafirlukast; dopamine receptor agonists such as cabergoline, bromocriptine, ropinirole and 4-hydroxy-7-[2-[[2-[[3-(2-phenylethoxy)-propyl]sulfonyl]ethyl]-amino]ethyl]-2(3H)-benzothiazolone and pharmaceutically acceptable salts thereof (the hydrochloride being Viozan^{®}- AstraZeneca); PDE4 inhibitors such as Ariflo^{®} (GlaxoSmith Kline), Roflumilast (Byk Gulden), enprofylline,V-11294A (Napp), BAY19-8004 (Bayer), D-4396 (Sch-351591), (Schering-Plough), Arofylline (Almirall Prodesfarma), PD189659 (Parke-Davis), AWD-12-281 (Asta Medica), BY343, CDC-801 (Celgene), CP-325366, and KW-4490 (Kyowa Hakko Kogyo) (including physiologically acceptable acid addition salts thereof such as salts of hydrochloric acid, hydrobromic acid, sulphuric acid, phosphoric acid, methanesulphonic acid, acetic acid, fumaric acid, succinic acid, lactic acid, citric acid, tartaric acid and maleic acid); A2a agonists such as those described in EP 409595A2, EP 1052264, EP 1241176, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, WO 03/086408, WO 04/039762, WO 04/039766, WO 04/045618 and WO 04/046083; and A2b antagonists such as those described in WO 02/42298.

Such bronchodilatory drugs include anticholinergic or antimuscarinic agents, in particular ipratropium bromide, oxitropium bromide, tiotropium salts, CHF 4226 (Chiesi), SVT-40776 and glycopyrrolate and other glycopyrronium salts, but also those described in EP 424021, US 3714357, US 5171744, US 2005/171147, US 2005/182091, WO 01/04118, WO 02/00652, WO 02/51841, WO 02/53564, WO 03/00840, WO 03/33495, WO 03/53966, WO 03/87094, WO 04/018422, WO 04/05285 and WO 05/077361.

Suitable dual anti-inflammatory and bronchodilatory drugs include dual beta-2 adrenoceptor agonist / muscarinic antagonists such as those disclosed in US 2004/0167167, US 2004/0242622, US 2005/182092, WO 04/74246 and WO 04/74812.

Suitable antihistaminic/anti-allergic drug substances include acetaminophen, activastine, astemizole, azelastin, bamipin, cetirizine hydrochloride, cexchloropheniramine, chlorophenoxamine, clemastine fumarate, desloratidine, dimenhydrinate, dimetinden, diphenhydramine, doxylamine, ebastine, emedastin, epinastine, fexofenadine hydrochloride, ketotifen, levocabastin, loratidine, meclizine, mizolastine, pheniramine, promethazine and tefenadine, as well as those disclosed in JP 2004107299, WO 03/099807 and WO 04/026841 (including any pharmacologically acceptable acid addition salts thereof which may exist).

Combinations of the compound of formula I in crystal form Qalpha and steroids, PDE4 inhibitors or LTD4 antagonists are particularly suitable for use in the treatment of asthma.

Combinations of the compound of formula I in crystal form Qalpha and anticholinergic or antimuscarinic agents, PDE4 inhibitors, LTB4 antagonists are particularly suitable for use in the treatment of COPD.

In accordance with the foregoing, the invention also provides a method for the treatment of an inflammatory condition, particularly an inflammatory or obstructive airways disease, which comprises administering to a subject, particularly a human subject, in need thereof an effective amount of the compound of formula I in crystal form Qalpha as hereinbefore described. In another aspect the invention provides the use of the compound of formula I in crystal form Qalpha for the manufacture of a medicament for the treatment of an inflammatory condition, particularly an inflammatory or obstructive airways disease.

The compound of formula I in crystal form Qalpha may be administered by any appropriate route, e.g. orally, for example in the form of a tablet or capsule; parenterally, for example intravenously; by inhalation, for example in the treatment of inflammatory or obstructive airways disease; intranasally, for example in the treatment of allergic rhinitis; topically to the skin, for example in the treatment of atopic dermatitis; or rectally, for example in the treatment of inflammatory bowel disease.

In a further aspect, the invention also provides a pharmaceutical composition comprising as active ingredient the compound of formula I in crystal form Qalpha optionally together with a pharmaceutically acceptable diluent or carrier therefor. The composition may contain a co-therapeutic agent such as a bronchodilatory or anti-inflammatory drug as hereinbefore described. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g. patches. Compositions for inhalation may comprise aerosol or other atomizable formulations or dry powder formulations.

When the composition comprises an aerosol formulation, it preferably contains, for example, a hydro-fluoro-alkane (HFA) propellant such as HFA134a or HFA227 or a mixture of these, and may contain one or more co-solvents known in the art such as ethanol (up to 20% by weight), and/or one or more surfactants such as oleic acid or sorbitan trioleate, and/or one or more bulking agents such as lactose. When the composition comprises a dry powder formulation, it preferably contains, for example, the compound of formula I in crystal form Qalpha having a particle diameter up to 10 microns, optionally together with a diluent or carrier, such as lactose, of the desired particle size distribution and a compound that helps to protect against product performance deterioration due to moisture e.g. magnesium stearate, typically 0.05-2.0% magnesium stearate. When the composition comprises a nebulised formulation, it preferably contains, for example, the compound of formula I in crystal form Qalpha either dissolved, or suspended, in a vehicle containing water, a co-solvent such as ethanol or propylene glycol and a stabiliser, which may be a surfactant.

The invention includes (A) the compound of formula I in crystal form Qalpha in inhalable form, e.g. in an aerosol or other atomisable composition or in inhalable particulate, e.g. micronised, form, (B) an inhalable medicament comprising the compound of formula I in crystal form Qalpha in inhalable form; (C) a pharmaceutical product comprising the compound of formula I in crystal form Qalpha in inhalable form in association with an inhalation device; and (D) an inhalation device containing the compound of formula I in crystal form Qalpha in inhalable form.

Dosages of the compound of formula I in crystal form Qalpha employed in practising the present invention will of course vary depending, for example, on the particular condition to be treated, the effect desired and the mode of administration. In general, suitable daily dosages for administration by inhalation are of the order of 0.005 to 10 mg, while for oral administration suitable daily doses are of the order of 0.05 to 100 mg.

The invention is illustrated by the following Examples.

### EXAMPLES

### Example 1

### Preparation of crystal form Qalpha

50 mg (R)-5-[2-(5,6-diethyl-indan-2-ylamino)-1-hydroxyethyl]-8-hydroxy-1H-quinolin-2-one maleate prepared by the process described in international patent application WO 05/123684 is equilibrated in 1 ml ethanol ALI (90% ethanol, 5% water, 5% isopropanol) over 3 days at 25 °C ± 0.1. The product is filtered and dried for 10 minutes in the air. The compound of formula I is obtained in the form of white crystals.

### Example 2

### Characterisation of crystal form Qalpha by X-ray powder diffraction

The X-ray diffraction pattern of crystal form Qalpha prepared in accordance with Example 1 is measured using a SCINTAG™ X-ray diffractometer with a CuK alpha radiation source. The X-ray diffraction pattern thus determined is shown in Figure 1 and represented in Table 1 below by the reflection lines and intensities of the most important lines.

**TABLE 1**

| **X-ray diffraction lines and intensities for crystal Qalpha** | | |
|---|---|---|
| **2θ (°)** | **d-spacings (Å)** | **Relative intensity** |
| 5.3 | 16.3 | Strong |
| 104.1 | 8.7 | Medium |
| 10.9 | 8.1 | Low |
| 11.3 | 7.8 | Low |
| 12.2 | 7.2 | Medium |
| 12.7 | 6.9 | Medium |
| 12.9 | 6.8 | Medium |
| 16.4 | 5.3 | Medium |
| 16.8 | 5.2 | Low |
| 17.8 | 4.9 | Low |
| 20.0 | 4.4 | Medium |
| 21.6 | 4.1 | Low |
| 22.0 | 4.0 | Low |
| 22.8 | 3.8 | Low |
| 23.4 | 3.7 | Medium |
| 23.6 | 3.7 | Low |
| 24.5 | 3.6 | Medium |
| 25.8 | 3.4 | Medium |
| 26.8 | 3.3 | Medium |
| 27.5 | 3.2 | Low |
| 27.9 | 3.1 | Low |
| 28.8 | 3.0 | Medium |
| 29.7 | 2.9 | Medium |
| 32.1 | 2.7 | Low |

The XRPD pattern shows a strong diffraction peak at 5.3°.

The X-ray diffraction pattern for amorphous (R)-5-[2-(5,6-diethyl-indan-2-ylamino)-1-hydroxyethyl]-8-hydroxy-1H-quinolin-2-one maleate obtained using the same diffractometer with alpha radiation source is shown in Figure 2.

### Example 3

### Characterisation of crystal form Qalpha by Raman spectroscopy

The FT-Raman spectrum of crystal form Qalpha prepared in accordance with Example 1 is measured using a BRUKER OPTICS RFS 100™ spectrometer. The FT-Raman spectrum thus determined is shown in Figure 3 and represented in Table 2 below by the reflection lines and intensities of the most important lines.

**TABLE 2**

| **FT-Raman spectral lines and intensities for crystal form Qalpha** | |
|---|---|
| Range/cm⁻¹ | cm⁻¹ |
| 3100-3000 | 3050, 2971, 2926, |
| | 2874 |
| 1700-1400 | 1691, 1665, 1619, |
| | 1568 |
| 1300-1000 | 1062 |
| 880-800 | 880-790 |
| 740-710 | (735) 711 |

The FT-Raman spectrum for amorphous (R)-5-[2-(5,6-diethyl-indan-2-ylamino)-1-hydroxyethyl]-8-hydroxy-1H-quinolin-2-one maleate obtained using the same spectrometer is shown in Figure 4.

### Example 4

### Characterisation of crystal form Qalpha by IR spectroscopy

The FT-IR spectrum of crystal form Qalpha prepared in accordance with Example 1 is measured using the transmission KBr technique and a BRUKER OPTICS IFS-55™ Fourier Transform Infrared (FTIR) spectrometer. The FT-IR spectrum thus determined is shown in Figure 5. Major IR bands are recorded at 3391, 3346, 2966, 2873, 1876, 1665, 1603, 1481, 1385, 1282, 1247, 1227, 1150, 1084, 1033, 872, 833, 657, 591 cm⁻¹.

The IR-Raman spectrum for amorphous (R)-5-[2-(5,6-diethyl-indan-2-ylamino)-1-hydroxyethyl]-8-hydroxy-1H-quinolin-2-one maleate obtained using the same spectrometer is shown in Figure 6.

### Example 5

### Characterisation of crystal form Qalpha by its crystallographic data

Crystal form Qalpha prepared in accordance with Example 1 has the crystallographic data given in Table 3 below.

**TABLE 3**

| **Crystallographic data for crystal form Qalpha** | |
|---|---|
| Cell content | C₅₆H₆₄N₄O₁₄ |
| fw | 508.56 |
| system | Triclinic |
| space group | P1 |
| a, Å | 8.867(2) |
| b, Å | 9.761(2) |
| c, Å | 16.696(2) |
| α, ° | 102.60(1) |
| β, ° | 94.19(1) |
| γ, ° | 113.24(2) |
| V, Å3 | 1275.3(4) |
| Z | 2 |
| calc, g/cm³ | 1.324 |
| R | 0.070 |

### Example 6

### Characterisation of crystal form Qalpha by its crystal-packing pattern

The crystal packing of Qalpha consists of (R)-5-[2-(5,6-diethyl-indan-2-ylamino)-1-hydroxyethyl]-8-hydroxy-1H-quinolin-2-one cations and maleate anions held together by H-bonds and electrostatic forces. Distances and angles of H-bonds have the characteristics shown in Table 4.

**TABLE 4**

| **Crystal-packing data for crystal form Qalpha** | | | | |
|---|---|---|---|---|
| Bond | Bond distance | | Bond | H-bond angle |
| O2 ... O72 | 2.601 Å | | O2-H ... O72 | 166° |
| O3 ...O1 | 2.683 Å | | O3-H ...O1 | 170° |
| N4 ... O62 | 2.760 Å | | N4-H ... O62 | 158° |
| N4 ... O61 | 2.810 Å | | N4-H ... O61 | 155° |
| O32 ... O60 | 2.665 Å | | O32-H ... O60 | 138° |
| O33 ... O3 | 2.835 Å | | O33-H ... O3 | 174° |
| N34 ... O70 | 2.787 Å | | N34-H ... O70 | 154° |
| N34 ... O73 | 2.835 Å | | N34-H ... O73 | 152° |
| N35 ... O33 | 2.962 Å | | N35-H ... O33 | 161° |
| O63 ... O61 | 2.472 Å | | O63-H ... O61 | 161° |
| O71 ... O73 | 2.431 Å | | O71-H...O73 | 155° |

Alternating columns of indane moieties and lines of quinolone fragments can be observed. From 12 available hydrogen atoms, 11 are involved in H-bonds. Only N5 is not participating.

### Example 7

### Characterisation of crystal form Qalpha by scanning electron microscopy

Samples of crystal form Qalpha prepared in accordance with Example 1 observed by scanning electron microscopy (SEM) reveal a population of large needle-shaped crystals.

## Claims

1. Crystalline (R)-5-[2-(5,6-diethyl-indan-2-ylamino)-1-hydroxyethyl]-8-hydroxy-1H-quinolin-2-one maleate designated crystal form Qalpha.

2. Crystalline (R)-5-[2-(5,6-diethyl-indan-2-ylamino)-1-hydroxyethyl]-8-hydroxy-1H-quinolin-2-one maleate designated crystal form Qalpha **characterised by** a melting point, by Differential Scanning Calorimetry, of about 192°C with simultaneous decomposition.

3. Crystalline (R)-5-[2-(5,6-diethyl-indan-2-ylamino)-1-hydroxyethyl]-8-hydroxy-1H-quinolin-2-one maleate designated in crystal form Qalpha having the following characteristic diffraction lines (20 in angular degrees ± 0.2°) in the X-ray diffraction pattern thereof: 5.3 °, 10.1 °, 12.7 °, 16.4 °, 20.0 ° and 25.8°.

4. Crystalline (R)-5-[2-(5,6-diethyl-indan-2-ylamino)-1-hydroxyethyl]-8-hydroxy-1H-quinolin-2-one maleate according to claim 3, that also has the following characteristic diffraction lines (2θ in angular degrees ± 0.2°) in the X-ray diffraction pattern thereof: 12.2 °, 12.9 °, 23.4 °, 24.5 °, 26.8 °, 28.8° and 29.7°.

5. Crystalline (R)-5-[2-(5,6-diethyl-indan-2-ylamino)-1-hydroxyethyl]-8-hydroxy-1H-quinolin-2-one maleate according to any one of claims 1 to 4 for use as a pharmaceutical.

6. A pharmaceutical composition comprising, as active ingredient, an effective amount of crystalline (R)-5-[2-(5,6-diethyl-indan-2-ylamino)-1-hydroxyethyl]-8-hydroxy-1H-quinolin-2-one maleate, optionally together with a pharmaceutically acceptable carrier.

7. A pharmaceutical composition according to claim 6, further comprising one, two, three or more anti-inflammatory, bronchodilatory, antihistaminic/anti-allergic or anti-tussive drug substances.

8. A pharmaceutical composition according to claim 6 or 7 , which is in inhalable form.

9. The use of crystalline (R)-5-[2-(5,6-diethyl-indan-2-ylamino)-1-hydroxyethyl]-8-hydroxy-1H-quinolin-2-one maleate for the preparation of a medicament for the treatment of an inflammatory or obstructive airways disease.

10. A method of preparing crystalline (R)-5-[2-(5,6-diethyl-indan-2-ylamino)-1-hydroxyethyl]-8-hydroxy-1H-quinolin-2-one maleate which comprises crystallising (R)-5-[2-(5,6-diethyl-indan-2-ylamino)-1-hydroxyethyl]-8-hydroxy-1H-quinolin-2-one maleate from a solution thereof in ethanol ALI (90% ethanol, 5% water, 5% isopropanol).

11. Crystalline (R)-5-[2-(5,6-diethyl-indan-2-ylamino)-1-hydroxyethyl]-8-hydroxy-1H-quinolin-2-one maleate designated crystal form Qalpha substantially as herein described with reference to any one or more of the Examples.
